(19) Europäisches Patentamt
European Patent Office
Office européen des brevets

(11) Veröffentlichungsnummer: **0 418 690 B1**

(12) **EUROPÄISCHE PATENTSCHRIFT**

(45) Veröffentlichungstag der Patentschrift: **22.03.95**

(21) Anmeldenummer: **90117367.4**

(22) Anmeldetag: **10.09.90**

(51) Int. Cl.$^{6}$: **C07C 403/16**, C07C 45/67, C07C 49/21, C07C 45/51, C07C 33/05, C07C 33/44, C07C 29/58

# (54) Verfahren zur Herstellung von Ironen.

(30) Priorität: **21.09.89 CH 3430/89**

(43) Veröffentlichungstag der Anmeldung:
**27.03.91 Patentblatt 91/13**

(45) Bekanntmachung des Hinweises auf die Patenterteilung:
**22.03.95 Patentblatt 95/12**

(84) Benannte Vertragsstaaten:
**CH DE ES FR GB IT LI NL**

(56) Entgegenhaltungen:
**CH-A- 373 034**
**FR-A- 1 393 451**

(73) Patentinhaber: **GIVAUDAN-ROURE (INTERNATIONAL) S.A.**
**CH-1214 Vernier, Genève (CH)**

(72) Erfinder: **Fráter, Georg, Dr.**
**Turicumstrasse 5**
**CH-8610 Uster (CH)**
Erfinder: **Helmlinger, Daniel, Dr.**
**Tichelrütistrasse 18**
**CH-8044 Gockhausen (CH)**

(74) Vertreter: **Urech, Peter, Dr. et al**
**Grenzacherstrasse 124**
**Postfach 3255**
**CH-4002 Basel (CH)**

**EP 0 418 690 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von α- und β-Iron, also den Verbindungen der Formel I, sowie neue Zwischenprodukte dieser Synthese.

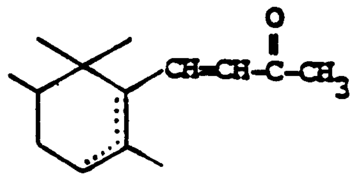

I

Das Verfahren ist dadurch gekennzeichnet, dass man das 8-(2′2′-Dimethylcyclopropyl)-6-methyl-4,5-octadien-2-on, also die Verbindung der Formel III

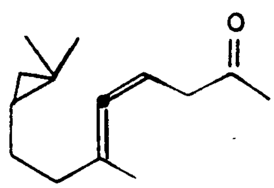

III

zu 8-( 2′2′-Dimethyl-cyclopropyl) -6-methyl-3,5-octadien -2-on, also der Verbindung der Formel II

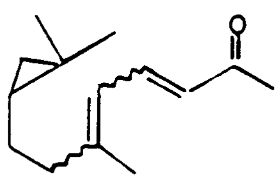

II

isomerisiert und hierauf II in an sich bekannter Weise in die Verbindung I überführt.

Die Isomerisierung von III kann sauer oder alkalisch durchgeführt werden.

Die Verbindung II, für welche Verbindung bisher keinerlei praktikabler Zugang existierte, entsteht üblicherweise als Gemisch der Isomeren

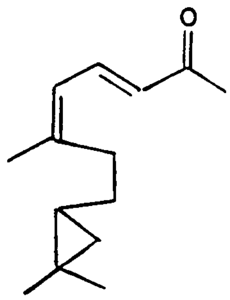

und

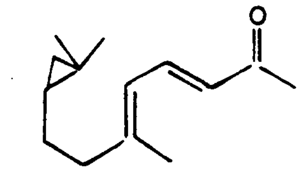

IIa (EZ)

IIb (EE)

Um im Endprodukt I einen möglichst hohen Anteil an cis-α-Iron, dem parfümistisch wertvolleren Isomeren, zu erzielen, isomerisiert man III vorzugsweise unter der Einwirkung einer Säure, oder man isomerisiert zunächst mit Base und behandelt anschliessend noch mit Säure. Auf diese Weise erzielt man einen hohen Anteil an benötigtem Isomeren II b.

Die Verbindung III wird zweckmässigerweise dadurch erhalten, dass man das 5-(2'2'-Dimethylcyclopropyl)-3-methyl-1-pentin-3-ol, also die Verbindung der Formel IV

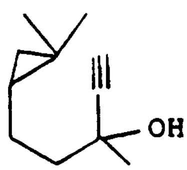

IV

in Gegenwart eines n-Alkylisopropenyläthers mit einer Säure behandelt. Dabei wird, für den Fachmann überraschenderweise, der Dreiring nicht angegriffen, d.h. es bildet sich als Reaktionsprodukt nur das Allenketon III.

Die Verbindung IV ist zugänglich aus einer Verbindung V

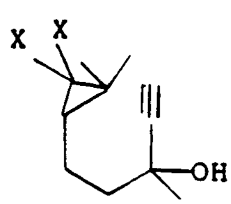

V

worin X für Chlor oder Brom steht,
und zwar durch Behandlung mit einem Alkalimetall in flüssigem Ammoniak.

Die Verbindung V kann aus Dehydrolinalool, also aus der Verbindung VI

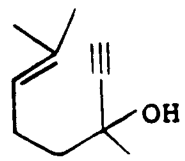

VI

durch Cycloaddition von Dihalogencarbenen erhalten werden.

Dabei wird, für den Fachmann überraschenderweise, nur die Doppelbindung, nicht aber auch die Dreifachbindung von VI angegriffen.

Die geeigneten Verfahrensparameter sind die folgenden:

| VI | V |
| --- | --- |
| Methodik | Bildung von Dihalogencyclopropanderivaten durch Cycloaddition von Dihalogencarbenen an Dehydrolinalool, wobei die Dihalogencarbene aus Haloform und einer starken Base mittels der Phasentransfermethode zugänglich sind (siehe z.B. Kleveland et al., Acta Chem. Scand. B. 31 (1977) 463-468) |
| Medium | organisch/wässrig-alkalisch (Chloroform, Bromoform),<br>Haloform/konzentrierte wässrige Laugen Phasentransferkatalysatoren, z.B. Trialkylbenzylammoniumhalogenide |
| Temperatur | ca. 15 - 35 °C, insbesondere ca. 15 - 20°C |
| Aufarbeitung | Extraktion mit chlorierten Kohlenwasserstoffen, z.B. Methylenchlorid |

V IV

| | |
|---|---|
| Methodik | nach den an sich bekannten Methoden von Dehydrohalogenierungen mittels Alkalimetallen in flüssigem Ammoniak, z.B. Li, Na, Org. Synthesis 54 (1974) 11 |
| Medium | flüssiges Ammoniak, V in einem Aether gelöst vorlegen, beispielsweise in Diäthyläther, Tetrahydrofuran, Dimethoxyäthan, aber auch Hexan, etc. kommt in Frage. |
| Temperatur | ca. -40 - -30 °C |
| Aufarbeitung | Abdampfen des $NH_3$, Zugabe eines Alkohols, z.B. Aethanol, zwecks Zerstören des überschüssigen Metalls, Ansäuern, Destillation |

| IV | III |
|---|---|
| Methodik | nach an sich bekannten Methoden zur Herstellung von ß-Ketoallenen aus Acetylencarbinolen mittels Vinyläthern in Gegenwart starker Säuren bei erhöhten Temperaturen (G. Saucy et al., Helv. Chim. Acta 50 (1967) 1158) |
| Medium | Petroläther, Hexan, Cyclohexan, etc. Mineralsäuren, z.B. Schwefelsäure, Phosphorsäure, etc. oder starke organische Säuren, wie p-Toluolsulfonsäure, etc. Vinyläther: n-Alkyl(Methyl, Aethyl, etc. )isopropenyläther |
| Temperatur | ca. 70° - 100 °C, unter Druck |
| Aufarbeitung | Extraktion mit organischen Lösungsmitteln, wie z.B. Aether oder Methylenchlorid, neutral waschen |

| III | II (sauer) |
|---|---|
| Methodik | nach den an sich bekannten Methoden der Isomerierung von Allenen in ein System konjugierter Doppelbindungen mittels Säuren. (I. Kuwajima et al. Tet. Lett. 21, (1980), 3209, T.F. Rutledge. Acetylenes and allenes, Reinhold Book Corporation (1968), 47). |
| Medium | 1. Alkohol, z.B. Methanol, Ethanol, etc. und/oder anderes organisches Lösungsmittel wie Toluol, Methylenchlorid, Aether, Dimethoxyäthan, etc. Bevorzugt: Alkohol und ein weiteres organisches Lösungsmittel.<br>2. Säure: starke organische Säure, z.B. p-Toluolsulfonsäure, Mineralsäure, z.B. Schwefelsäure, saurer Ionentauscher, z.B. Amberlite IR 120, etc. |
| Temperatur | Temperaturen um Raumtemperatur sind bevorzugt. |
| Aufarbeitung | Auf Wasser giessen, mit organischem Lösungsmittel (z.B. Aether) extrahieren, neutral waschen, eindampfen. |

| III | II (basisch) |
| --- | --- |
| Methodik | nach den an sich bekannten Methoden der Isomerierung von Allenen in ein System konjugierter Doppelbindungen mittels Basen (G. Saucy, R. Marbet, Helv. Chim. Acta 50 (1967) 1158 |
| Medium | Alkohol, z.B. Aethanol, Methanol / starke wässrige Base, z.B. ca. 30% bis 50% NaOH, ca. 30% bis 50% KOH, etc. |
| Temperatur | ca. 0 - 10°C |
| Aufarbeitung | Neutralisieren, Alkohol abdampfen. Extrahieren mit Aether oder Hexan, neutralwaschen, destillieren. |

| II | I |
|---|---|
| Methodik | Cyclisierung mittels Säure, (D. Felix et al., Chimia <u>18</u> (1964) 174;<br><br>US-PS 3,649,694; hier sind sämtliche relevanten Verfahrensparameter abgehandelt) |
| Medium | Mineralsäuren, wie Schwefelsäure, Phosphorsäure, Gemische von Schwefelsäure und Essigsäure.<br>Lewissäuren wie Bortrifluorid, Aluminiumchlorid, etc.<br>Lösungsmittel fakultativ, zum Beispiel (Diethyl) -Aether, Benzol, Toluol, Methylenchlorid, Aethylenchlorid, etc. |

Die Verbindungen der Formel I können in der cis- und trans-Form vorliegen [geometrische Isomerie in der Seitenkette; Stereochemie am Ring]. Im Falle der Stereochemie am Ring sollen beide Formen umfasst werden, im Falle der Isomerie in der Seitenkette die trans-Form. Im Falle der Verbindung der Formel II soll die Formel die trans-Stellung in 3-Stellung und die cis- und trans-Form in 5-Stellung umfassen.

Das Iron fällt als Gemisch von cis α-, trans α-und β-Iron an. Eine Auftrennung in die einzelnen Isomeren ist zwar möglich, für die Verwendung als Riechstoff meistens gar nicht erforderlich. Als Trennungsmethodik eignet sich beispielsweise die Destillation (z.B. mittels Drehbandkolonne, Sulzerkolonne, etc.) oder die Chromatographie.

Wie vorne ausgeführt, ist ein Gemisch mit einem möglichst hohen Anteil an cis-α-Iron bevorzugt.

Die einzelnen Irone können durch folgende Formeln dargestellt werden:

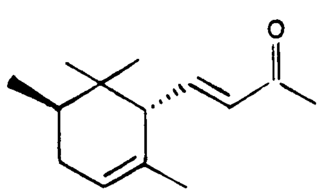

trans-α-Iron

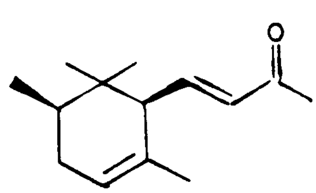

cis-α-Iron

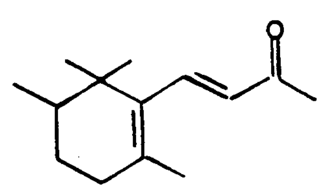

β-Iron

Beispiel 1

400 g (2,6 Mol) Dehydrolinalool und 5,26 g Triethylbenzylammoniumchlorid werden in einen Kolben vorgelegt. 469,6 g (3,9 Mol) Chloroform werden zugetropft. Zu dieser Lösung werden unter Eiskühlung innerhalb von 50 Minuten 223,2 g (2,79 Mol) Natronlauge 50% zugetropft, wobei die Temperatur des Reaktionsgemisches auf 34° steigt. Die anfänglich zähe Masse wird mit der Zeit dünnflüssiger. Nach 21 Stunden und dann nochmals 37 Stunden Rühren werden jeweils innert 20 Minuten unter Eiskühlung 160 ml Chloroform, 2,63 g Triethylbenzylammoniumchlorid und 111,6 g 50% NaOH zugegeben. Es wird noch 60 Stunden bei Raumtemperatur weiter gerührt, auf Eiswasser gegossen, mit Methylenchlorid extrahiert, neutralgewaschen, über Magnesiumsulfat getrocknet und eingedampft. Man erhält auf diese Weise 603,7 g (97%) 5-( 2′2′-Dichloro-3′3′-dimethylcyclopropyl)-3-methyl-1-pentin-3-ol.
IR (Film): 3300;
H-NMR ($CDCl_3$, 400MHz) 1,2 (s, $CH_3$-C(2′));1,35 (s, $CH_3$-C(2′)); 1,52 (s, $CH_3$-C(3)); 2,47 (s, H-C(1));
MS: 181 (13,3); 163 (6,6); 139 (29,6); 137 (26,6); 115 (9,6); 91 (15,5); 79 (29,6); 69 (97); 59 (50); 43 (100); 27 (8,1);

Beispiel 2

60,8 g (0,4 Mol) Dehydrolinalool, 151,6 g (0,6 Mol) Bromoform und 0,8 g Triethylbenzylammoniumchlorid werden in einen Kolben vorgelegt, und es werden während 30 Minuten 64 g 50% Natronlauge zugetropft. Die Reaktion ist exotherm, man kühlt deshalb mit Eis.

Man rührt 21,5 Stunden bei Raumtemperatur nach und gibt nochmals die selbe Mengen Bromoform und Triethylbenzylammoniumchlorid zu. Die Reaktion ist nun exotherm. Man kühlt mit Eis und lässt noch 4 Stunden bei 0° nachreagieren. Das Reaktionsgemisch wird auf Eis gegossen. Es wird mit Methylenchlorid extrahiert. Die organischen Phasen werden neutral gewaschen, getrocknet und eingedampft. Man erhält auf diese Weise 109,5 g (84%) 5-( 2′2′-Dibrom-3′3′-dimethylcyclopropyl)-3-methyl-1-pentin-3-ol.
IR (Film): 3300;
H-NMR ($CDCl_3$, 400 MHz) 1,22 (s, $CH_3$-C(2′));1,4 (s, $CH_3$-C(2′); 1,54 (s, $CH_3$-C(3));2,48 (H-C(1)));
MS: 227 (11,8); 185 (4,4); 163 (11,8); 146 (48,1), 131 (31,1); 91 (16,2); 79 (27,4); 69 (50,3); 55 (21,4), 43 (100);

Beispiel 3

1 l Ammoniak wird in einen Kolben vorgelegt, 150 g (0,63 Mol) 5-( 2′2′-Dichloro-3′3′-dimethylcyclopropyl)-3-methyl-1-pentin-3-ol gelöst in 750 ml Aether werden zugetropft. Bei -30° bis -35° werden innerhalb von 2 1/2 Stunden 62 g (2,7 Mol) Natrium in kleinen Portionen bis zur bleibenden Blaufärbung zugegeben. Das Ammoniak wird abgedampft und 1,5 Liter Hexan werden zugetropft, und nachträglich werden noch 100 ml Aethanol zugegeben. Das Reaktionsgemisch wird auf ein Gemisch von 150 ml Essigsäure in 500 g Eis gegossen, nacheinander mit Wasser, gesättigter Natriumcarbonatlösung, gesättigter Kochsalzlösung neutral gewaschen, getrocknet und eingedampft. Das Rohprodukt (100,2 g) wird destilliert; man erhält auf diese Weise 90,5 g (85%) 5-(2′2′-Dimethylcyclo-propyl) 3-methyl-1 -pentin-3-ol.

54,5 ml Ammoniak werden in einen Kolben vorgelegt und 109,5 g (0,338 Mol) 5-(2′2′-Dibrom-3′3′-dimethylcyclopropyl) 3-methyl-1-pentin-3-ol, gelöst in 415 ml Diethyläther zugegeben. Bei -42° bis - 36° werden innerhalb von 1 1/2 Stunden 37,4 g (1,662 Mol) Natrium in kleinen Portionen zugegeben. Man rührt 1 3/4 h nach. Das Ammoniak wird abgedampft und 830 ml Hexan werden zugetropft. Man rührt 30 Minuten nach und tropft 60 ml Aethanol zu. Es wird nun auf ein Gemisch von Eis und Essigsäure gegossen, mit Aether extrahiert, neutral gewaschen und eingedampft. Das Rohprodukt (52,9 g) wird bei 0,06 Torr. destilliert. Auf diese Weise erhält man 27,4 g (48%) 5-( 2′2′-Dimethylcyclopropyl)-3-methyl-1-pentin-3-ol (Reinheit 75%).
IR (Film): 3300;
H-NMR ($CDCl_3$ 400 MHz) -0,1-(-0,04)(m,H-C(3)); 0,38-0,41 (m, H-C(3)); 0,44-0,54 (H-C(1)); 1,04 (s, $CH_3$-C-(2)); 1,07 (s, $CH_3$-C(2)); 1,5 (s, $CH_3$-C(3)); 2,43 (s, H-C(1));
MS: 151 (0,7); 133 (6); 123 (3,5); 109 (9,8); 105 (11,9); 91 (17,6); 82 (60,5); 69 (55); 67 (59,8); 55 (57); 41 (100); 29 (17,6).

Beispiel 4

Man gibt 30 g (0,18 Mol) 5-( 2'2'-Dimethylcyclopropyl)-3-methyl-1-pentin-3-ol, 37,8 g (0,52 Mol) Isopropenylmethyläther, 70 ml Isooctan, 40 mg Hydrochinon und 30 mg p-Toluolsulfonsäure in einen Kolben. Man erhitzt 24 Stunden unter Rühren (Oelbad 85°). Die Innentemperatur, die anfänglich 56° beträgt, steigt dabei auf 66°. Man gibt 10 mg p-Toluolsulfonsäure zu und erhitzt zwei weitere Stunden. (Oelbad 92°). Die Innentemperatur steigt auf 76°. Für die Gewinnung des 8-( 2',2'-Dimethylcyclopropyl)-6-methyl-3,5-octadien-2-ons kann dieses Reaktionsgemisch in der folgenden Stufe eingesetzt werden.

Zwecks Isolierung des Allens wird am Rollverdampfer unter Zugabe von Natriumbicarbonat eingedampft, mit Hexan verdünnt und mit gesättigter Kochsalzlösung gewaschen und eingedampft. Auf diese Weise erhält man 37 g (99%) rohes Allen, nämlich 8-(2'2'-Dimethylcyclopropyl)-6-methyl- 4,5-octadien-2-on, das noch zusätzlich durch Destillation gereinigt werden kann.

IR (Film:) 1720, 1960

H-NMR: (400 MHz ($CDCl_3$) - 0,107 (dd J(3'1') = 5, J(3'3') = 4Hz : H-C(3') trans zu H-C(1'));
0,36 (dd, J(3'1') = 8,5, J(3'3') = 4 H-C(3') cis zu H-C(1'));
0,44 (dddd J(1'3') = 8,5, J(3'1') = 5 J(1'8) = 7 J(1'8) = 7 : H-C(1'));
1,01 (s, $CH_3$-C(2')); 1,03 (s, $CH_3$-C(2')); 1,2-1,58 (m, $H_2$-C-(8));1,7 (d, J = 3, $CH_3$-C(6)); 2,02 (ddd, J = 8, J = 8, J = 3, $H_2$-C(7));
2,18 (s, $CH_3$-C(2)); 3,06 (d, J = 7, $H_2$-C(3));5,05-5,21 (m, H-C(4));

MS: 191 (2); 163 (2); 149 (2); 133 (6); 123 (50); 107 (37,3); 95 (16); 91 (20); 79 (33,8); 67 (18,3); 55 (62,6); 43 (100); 29 (20,4);

Beispiel 5

In eine Lösung von 2,5 ml 30-proz. Natronlauge in 32,5 ml Methanol lässt man bei 0-6°C innerhalb 45 Minuten das rohe Reaktionsgemisch des Beispiels 4 fliessen. Man rührt 10 Minuten nach. Es wird mit 1,45 ml Essigsäure neutralisiert und das Methanol wird am Rotationsverdampfer abdestilliert. Der Rückstand wird mit Wasser, gesättigter Natriumcarbonatlösung und gesättigter Kochsalzlösung gewaschen, getrocknet und eingedampft. Man destilliert und erhält auf diese Weise 3,2 g Ausgangsmaterial und 25,3 g (76% bezüglich des umgesetzten Edukts) 8-(2'2'-Dimethylcyclopropyl)-6-methyl-3,5-octadien-2-on (Sdp 83-95 / 0,06 Torr.) als EZ- und EE-Isomere im Verhältnis 2:1.

EZ - Isomeres:

IR (Film) 1590, 1625, 1665, 1690

H-NMR: -0,07 (dd J(3'1') = 5, J(3'3') = 4Hz : H-C(3') trans zu H-C(1'));
0,4 (dd, J(3'1') = 8,5, J(3'3') = 4, H-C(3') cis zu H-C(1'));
0,48 (dddd J(1'3') = 8,5, J(3'1') = 5, J(1'8) = 7, J(1'8) = 7 : H-C (1'));
1,02 (s, $CH_3$-C(2')); 1,07 (s, $CH_3$-C(2')); 1,47 (dd, J = 7; $H_2$-(C8); 1,9 (s, $CH_3$-(C6)); 2,27 (s, $CH_3$-C(2)); 2,38 (dd, J = 8, $H_2$-C(7)); 6,02 (d, J = 12, H-C(5)); 6,06 (d, J = 16, H-C(3)); 7,46 (dd, J = 16, J = 11, H-C(4));

$_{13}$C-NMR: 15,67 (s); 19,73 (t); 19,91 (q); 24,29 (d); 24,64 (q); 27,35 (q); 27,49 (q); 28,82 (t); 33,40 (t); 124,47 (d); 128,28 (d); 139,49 (d); 151,62 (s); 198,60 (s);

MS: 206 (2,1); 191 (3,5); 163 (5,6); 148 (4,9); 135 (6,3); 121 (11,2) 109 (74); 81 (68); 55 (100); 43 (85) 27 (19);

EE - Isomeres: IR (Film) 1590, 1630, 1670, 1690

H-NMR: 0,1 (dd) J(3'1') = 5, J(3'3') = 4 : H-C(3') trans zu H-C(1'));
0,38 (dd, J(3'1') = 8,5, J(3'3') = 4 : H-C(3') cis zu H-C(1'));
0,44 (dddd J(1'2') = 8,5, J(3'1') = 5, J(1'8') = 7, J(1'8') = 7, H-C(1');
1,01 (s, $CH_3$-C(2')); 1,04 (s, $CH_3$-C(2')); 1,36-1,54 (m, $H_2$-C(8)); 1,9 (d, J = 1Hz, $CH_3$-C(6)); 2,21 (dd, J = 8, H2-C (7); 2,25 (s, $CH_3$-C(2)); H-C(5)); 6,02 (d, J = 11, H-C(5)); 6,07 (d, J = 16, H-C(3)); 7,42 (dd, J = 16, J = 11, H-C( 4));

C-NMR: 15,57 (s); 17,59 (q); 19,73 (t); 19,92 (q); 24,30 (d), 27,50 (q); 27,52 (q); 28,27 (t); 41,00 (t); 123,67 (d); 128,34 (d); 139,69 (d); 151,60 (s); 198,80 (s);

MS: 206 (1,4); 191 (2,8); 163 (5,6); 148 (3,5); 135 (5,6); 122 (9,8); 109 (57); 91 (7,7); 82 (31,6); 67 (10,5); 55 (100); 43 (55); 29 (14);

Beispiel 6

5 g rohes 8-(2′,2′-Dimethylcyclopropyl)-6-methyl-3,5-octadien-2-on (Beispiel 5, EZ / EE = 2/1) wird in einem Gemisch von 6,5 ml Methanol und 5,7 ml Toluol gelöst und es werden 100 mg Paratoluolsulfonsäure zugegeben. Man rührt 17 Stunden bei Raumtemperatur. Es wird mit Toluol extrahiert, mit Wasser und gesättigter Kochsalzlösung neutral gewaschen, getrocknet und eingedampft. Man erhält auf diese Weise 4,8 g (96%) rohes 8-(2′,2′-Dimethylcyclopropyl-6-methyl-3,5-octadien-2-on, EZ / EE = 4/6.

Beispiel 7

4,5 g rohes 8-(2′,2′-Dimethylcyclopropyl)-6-methyl-3,5-octadien-2-on (Beispiel 6, EZ / EE = 4/6) werden in 26 ml Toluol gelöst und dann auf -4°C abgekühlt. Bei -4°C bis 4°C (exotherm) wird Bortrifluorid eingeleitet und zwar bis zur Sättigung der Lösung (Aufnahme: ca. 2 g Bortrifluorid). Dann wird unter schwachem Argonstrom zunächst 10 Minuten bei -2°C, dann 1 1/2 Stunden bei 10° gerührt. Es wird mit Aether extrahiert, mit Wasser und gesättigter Natriumcarbonatlösung neutral gewaschen, getrocknet und eingedampft. Man erhält auf diese Weise nach Kugelrohrdestillation 3,4 g (75%) eines Gemisches von trans-α-Iron, cis-α-Iron und β-Iron im Verhältnis 47/41/10.

Aus dem Gemisch kann nötigenfalls durch Destillation an einer Drehbandkolonne das höher siedende β-Iron abgetrennt werden.

Beispiel 8

5 g 8-(2′,2′ Dimethylcyclopropyl)-6-methyl-4,5-octadien-2 -on werden in einem Gemisch von 6 ml Methanol und 6 ml Toluol gelöst, dann werden 200 mg p-Toluolsulfonsäure zugegeben. Man rührt 6 3/4 Stunden bei Raumtemperatur. Es wird mit Aether verdünnt, mit gesättigter Bicarbonat- und Carbonat-Lösung und mit gesättigter Kochsalzlösung neutral gewaschen, getrocknet und eingeengt. Das Rohprodukt (4,5 g) wird kugelrohrdestilliert. Man erhält auf diese Weise 4,2 g (84%) 8-( 2′,2′ Dimethyl-cyclopropyl)-6-methyl-3,5-octadien-2-on. EZ / EE = 4/6.

## Patentansprüche

**1.** Verfahren zur Herstellung von α- und β-Iron, dadurch gekennzeichnet, dass man 8-(2'2'-Dimethylcyclopropyl)-6-methyl-4,5-octadien-2-on zu 8-(2'2'-Dimethylcyclopropyl)-6-methyl-3,5-octadien-2-on isomerisiert und dieses Keton sauer zu α- und β-Iron cyclisiert.

**2.** Verfahren nach Anspruch 1,
dadurch gekennzeichnet, dass man mittels Base, z.B. einem Alkalimetallhydroxyd, oder, vorzugsweise, mittels Säure, z.B. einer starken organischen Säure, isomerisiert.

**3.** Verfahren nach Anspruch 1 oder 2,
dadurch gekennzeichnet, dass man das 8-( 2'2'-Dimethylcyclopropyl)-6-methyl-4,5-octadien-2-on durch Behandlung von 5-( 2'2'-Dimethylcyclopropyl)-3-methyl-1-pentin-3-ol mit einem n-Alkylisopropenyläther in Gegenwart einer Säure erhält.

**4.** Verfahren nach Anspruch 3,
dadurch gekennzeichnet, dass man die Reaktion bei einer Temperatur von ca. 70-100°C durchführt.

**5.** Verfahren nach einem der Ansprüche 1-4,
dadurch gekennzeichnet, dass man das 5-(2',2'-Dimethylcyclopropyl)-3-methyl-1-pentin-3-ol durch Enthalogenierung von 5-(2'2'-Dihalogen-3',3'dimethylcyclopropyl)-3-methyl-1-pentin-3-ol gewinnt.

**6.** Verfahren nach Anspruch 5,
dadurch gekennzeichnet, dass man mittels eines Alkalimetalls in flüssigem Ammoniak reduziert.

**7.** Verfahren nach einem der Ansprüche 1-6, dadurch gekennzeichnet, dass man das β-Iron jeweils abtrennt, d.h. entfernt.

**8.** 8-(2'2'-Dimethylcyclopropyl)-6-methyl-4,5-octadien-2-on.

**9.** 5-(2'2'-Dimethylcyclopropyl)-3-methyl-1-pentin-3-ol.

**10.** 5-(2'2',-Dichlor-3',3'-dimethylcyclopropyl)3-methyl-1-pentin-3-ol.

**11.** 5-(2'2'-Dibrom-3',3'-dimethylcyclopropyl)-3-methyl-1-pentin-3-ol.

## Claims

**1.** A process for the manufacture of α- and β-irone, characterized by isomerizing 8-(2'2'-dimethylcyclopropyl)-6-methyl-4,5-octadien-2-one to 8-(2'2'-dimethylcyclopropyl)-6-methyl-3,5-octadien-2-one and acidically cyclizing this ketone to α- and β-irone.

**2.** A process according to claim 1, characterized in that the isomerization is carried out using a base, e.g. an alkali metal hydroxide, or, preferably, using an acid, e.g. a strong organic acid.

**3.** A process according to claim 1 or 2, characterized in that the 8-(2'2'-dimethylcyclopropyl)-6-methyl-4,5-octadien-2-one is obtained by treating 5-(2'2'-dimethylcyclopropyl)-3-methyl-1-pentyn-3-ol with a n-alkyl isopropenyl ether in the presence of an acid.

**4.** A process according to claim 3, characterized in that the reaction is carried out at a temperature of about 70-100 °C.

**5.** A process according to any one of claims 1-4, characterized in that the 5-(2,2'-dimethylcyclopropyl)-3-methyl-1-pentyn-3-ol is obtained by dehalogenating 5-(2'2'-dihalo-3',3'-dimethylcyclopropyl)-3-methyl-1-pentyn-3-ol.

**6.** A process according to claim 5, characterized in that reduction is carried out using an alkali metal in liquid ammonia.

**7.** A process according to any one of claims 1-6, characterized in that the β-irone is separated, i.e. removed.

**8.** 8-(2'2'-Dimethylcyclopropyl)-6-methyl-4,5-octadien-2-one.

**9.** 5-(2'2'-Dimethylcyclopropyl)-3-methyl-1-pentyn-3-ol.

**10.** 5-(2'2'-Dichloro-3',3'-dimethylcyclopropyl)-3-methyl-1-pentyn-3-ol.

**11.** 5-(2'2'-Dibromo-3',3'-dimethylcyclopropyl)-3-methyl-1-pentyn-3-ol.

## Revendications

**1.** Procédé pour la préparation des α- et β-irones, caractérisée en ce que l'on isomérisé la 8-(2',2'diméthylcyclopropyl)-6-méthyl-4,5-octadién-2-one en 8-(2',2'--diméthylcyclopropyl)-6-méthyl-3,5-octadién-2-one et en ce que l'on cyclise par voie acide cette cétone en α- et β-irones.

**2.** Procédé selon la revendication 1, caractérisé en ce que l'on isomérise par une base, par exemple par un hydroxyde de métal alcalin ou, de préférence, par un acide, par exemple par un acide organique fort.

**3.** Procédé selon la revendication 1 ou 2, caractérisé en ce que l'on obtient la 8-(2',2'-diméthylcyclopropyl)-6-méthyl-4,5-octadién-2-one par traitement du 5-(2',2'-diméthylcyclopropyl)-3-méthyl-1-pentin-3-ol avec un éther n-alkylisopropénylique en présence d'un acide.

**4.** Procédé selon la revendication 3, caractérisé en ce que l'on effectue la réaction à une température allant d'environ 70 à environ 100 °C.

5. Procédé selon l'une des revendications 1 à 4, caractérisé en ce que l'on obtient le 5-(2',2'-diméthylcyclopropyl)-3-méthyl-1-pentin-3-ol par déshalogénation du 5-(2',2'-dihalogéno-3',3'-diméthylcyclopropyl)-3-méthyl-1-pentin-3-ol.

6. Procédé selon la revendication 5, caractérisé en ce que l'on réduit par un métal alcalin dans l'ammoniac liquide.

7. Procédé selon l'une des revendications 1 à 6, caractérisé en ce que l'on sépare la $\beta$-irone, c'est-à-dire en ce qu'on l'élimine.

8. La 8-(2',2'-diméthylcyclopropyl)-6-méthyl-4,5-octadién-2-one.

9. Le 5-(2',2'-diméthylcyclopropyl)-3-méthyl-1-pentin-3-ol.

10. Le 5-(2'2'-dichloro-3',3'-diméthylcyclopropyl)-3-méthyl-1-pentin-3-ol.

11. Le 5-(2',2'-dibromo-3',3'-diméthylcyclopropyl)-3-méthyl-1-pentin-3-ol.